(19) Europäisches Patentamt
European Patent Office
Office européen des brevets

(11) Numéro de publication: **0 012 096**
**B1**

(12) **FASCICULE DE BREVET EUROPEEN**

(45) Date de publication du fascicule du brevet: **24.03.82**

(51) Int. Cl.³: **C 07 D 307/86**

(21) Numéro de dépôt: **79420062.6**

(22) Date de dépôt: **28.11.79**

(54) **Procédé de préparation du dihydro-2,3 diméthyl-2,2 hydroxy-7 benzofurane.**

(30) Priorité: **30.11.78 FR 7834296**
**18.07.79 FR 7919126**

(43) Date de publication de la demande:
**11.06.80 Bulletin 80/12**

(45) Mention de la délivrance du brevet:
**24.03.82 Bulletin 82/12**

(84) Etats contractants désignés:
**AT BE CH DE FR GB IT LU NL SE**

(56) Documents cités:
**FR - A - 1 403 952**
**FR - A - 1 548 441**

(73) Titulaire: **RHONE-POULENC AGROCHIMIE**
**14-20, rue Pierre Baizet**
**F-69009 Lyon (FR)**

(72) Inventeur: **Michelet, Daniel**
**24, Chemin de Montribloud**
**F-69160 Tassin la Demi Lune (FR)**
Inventeur: **Rakoutz, Michel**
**6, Boulevard de l'Europe**
**F-69600 Oullins (FR)**

(74) Mandataire: **Chaumette, Michel et al,**
**RHONE-POULENC AGROCHIMIE BP 9163-09**
**F-69263 Lyon Cedex 1 (FR)**

**EP 0 012 096 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Procédé de préparation du dihydro-2,3 diméthyl-2,2-hydroxy-7 benzofurane

L'invention concerne un procédé de préparation du dihydro-2,3 diméthyl-2,2 hydroxy-7 benzofurane.

Ce composé a pour formule:

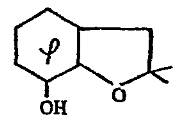

Le dihydro-2,3 diméthyl-2,2 hydroxy-7-benzofurane, désigné dans ce qui suit par DDHB, est un composé en soi connu, utilisable pour la préparation du méthylcarbamate de dihydro-2,3 diméthyl-2,2 benzofuranyl-7, insecticide polyvalent connu sous le nom de carbofuran.

Il est connu, d'après le brevet américain 3 474 171, que le DDHB peut être obtenu à partir d'orthométhallyloxyphénol, par réarrangement de ce composé (transposition ortho du radical méthallyle et cyclisation du composé ainsi formé. Selon ce brevet, le DDHB est obtenu par chauffage dans la masse de l'orthométhallyloxyphénol sans utilisation de solvant ni de diluant.

L'orthométhallyloxyphénol est le composé de formule:

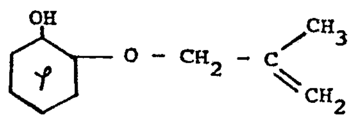

Par ailleurs, il est connu, d'après le brevet français FR - A - 1548 441, de préparer des dihydro-2,3 dialkyl-2,2 alkoxy-7 benzofuranes à partir d'alkoxy-1 alkényloxy-2 benzènes, par transposition en ortho du radical alkényle, puis cyclisation du composé intermédiairement formé. Selon ce brevet, la transposition/cyclisation peut s'effectuer en une seule étape, en présence du chlorure de magnésium anhydre, soit en deux étapes successives et la cyclisation s'effectue alors en présence d'un agent de cyclisation tel que l'acide formique, l'acide phosphorique, un mélange d'acide bromhydrique et d'acide acétique, et le le chlorure de magnésium. Le dihydro-2,3 dialkyl-2,2 alkoxy-7 benzofurane ainsi obtenu est ensuite transformé en le dihydro-2,3 dialkyl-2,2 hydroxy-7 benzofurane correspondant, par traitement dans un étape supplémentaire au moyen d'un chlorhydrate de pyridine.

Un but de la présente invention est de fournir un procédé amélioré de préparation du DDHB, notamment à partir d'orthométhallylpyrocatéchine.

Un autre but de l'invention est de préparer le DDHB à partir d'orthométhallyloxyphénol avec un rendement amélioré.

Un autre but de l'invention est de permettre de préparer essentiellement, en une seule étape, le DDHB à partir d'orthométhallyloxyphénol sans avoir à isoler le composé formé immédiatement.

Il a maintenant été trouvé que ces buts pouvaient être atteints grâce à un nouveau procédé qui fait l'objet de la présente invention.

Ce procédé est un procédé de préparation de DDHB à partir d'orthométhallylpyrocatéchine caractérisé en ce qu'on chauffe de l'orthométhallylpyrocatéchine en présence d'eau liquide et éventuellement d'un solvant organique inerte (liquide).

Par solvant inerte on entend un solvant n'entrant pas en réaction chimique dans le milieu réactionnel et dans les conditions opératoires.

Dans le présent exposé, par orthométhallylpyrocatéchine, on désigne le produit de formule:

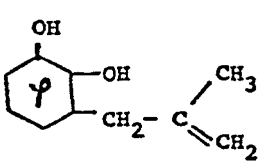

Selon une variante de l'invention l'orthométhallylpyrocatéchine est préparée par chauffage d'orthométhallyloxyphénol, en présence d'eau et/ou d'un solvant organique inerte.

Il s'en suit que, lorsque l'orthométhallylpyrocatéchine est préparée par chauffage, en présence d'eau, d'orthométhallyloxyphénol, alors on réalise un procédé (qui fait partie de l'invention) qui consiste (en une seule étape) à préparer du DDHB par chauffage d'orthométhallyloxyphénol en présence d'eau (liquide) et éventuellement d'un solvant organique inerte (liquide).

Il s'en suit aussi que, lorsque l'orthométhallylpyrocatéchine est préparée par chauffage, en présence d'un solvant organique inerte (liquide), d'orthométhallyloxyphénol, alors on réalise un procédé (qui fait partie de l'invention) et qui consiste à préparer du DDHB par chauffage d'orthométhallyloxyphénol et, pendant une partie de la durée de ce chauffage (en fin de chauffage), le milieu réactionnel contient de l'eau (liquide) et éventuellement un solvant organique inerte (liquide).

Un objet particulier de l'invention est donc un procédé de préparation de DDHB à partir d'orthométhallyloxyphénol, caractérisé en ce qu'on chauffe de l'orthométhallyloxyphénol et que, pendant tout ou partie de la durée de ce chauffage, le milieu réactionnel contient de l'eau (liquide) et éventuellement un solvant inerte (liquide). Selon un mode particulier de réalisation de l'invention, le chauffage de l'orthométhallylpyrocatéchine et/ou de l'orthométhallyloxyphénol sont réalisés simplement dans l'eau sans utilisation de solvant. Selon un autre mode de réalisation de l'invention, ces mêmes réactions sont effectuées en présence d'eau et d'un solvant organique inerte (liquide), c'est à dire ne réagissant pas chimiquement dans les conditions de la réaction.

Lorsqu'on prépare le DDHB par chauffage d'orthométhallylpyrocatéchine en présence d'eau et que, préalablement, l'orthométhallylpyrocatéchine est préparée par chauffage en présence d'eau d'orthométhallyloxyphénol, alors cette dernière réaction est effectuée en présence d'un solvant organique. Autrement dit encore, selon une variante de l'invention où le DDHB est préparée à partir d'orthométhallyloxyphénol, l'eau est présente dans le milieu réactionnel pendant toute la durée de mise en oeuvre du procédé l'invention; cette variante représente donc un procédé de préparation de DDHB à partir d'orthométhallyloxyphénol en présence d'eau, cette eau étant présente dès le début du chauffage.

Selon une autre variante de l'invention où le DDHB est préparé à partir d'orthométhallyloxyphénol, l'eau n'est introduite dans le milieu réactionnel qu'après une certaine période de chauffage au cours de laquelle l'orthométhallyloxyphénol chauffé sans eau et en présence d'un solvant organique inerte s'est transformé en tout ou partie en produits intermédiaires (orthométhallylpyrocatéchine notamment) et ce n'est qu'après cette première transformation que le milieu réactionnel contient de l'eau, ou, autrement dit, que le milieu réactionnel est chauffé en présence d'eau ou, encore autrement dit, que l'on ajoute de l'eau au milieu réactionnel.

Ces produits intermédiaires sont, notamment, une ou des méthallylpyrocatéchine(s).

La proportion relative d'eau et de solvant organique peut varier dans de grandes limites. De préférence, le rapport en volume de la quantité de solvant organique à la quantité d'eau utilisée est inférieur à 3.

La quantité d'eau mise en oeuvre par rapport à l'orthométhallylpyrocatéchine ou à l'orthométhallyloxyphénol engagé dans la réaction est généralement compris entre 0,1 et 20, de préférence entre 0,3 et 5 (rapport de quantités pondérales).

Le solvant organique peut être soit miscible à l'eau, soit non miscible à l'eau. Comme solvant organique, on utilise de préférence un solvant de point d'ébullition élevé choisi parmi les hydrocarbures aromatiques tels que le toluène, les o-, m- et p-xylènes; les hydrocarbures aromatiques chlorés tels que le chlorobenzène; les hydrocarbures aliphatiques tels que l'octane ou le dodécane; les hydrocarbures aliphatiques chlorés tels que le dichloro-1,2 éthane ou le trichloro-1,1,2 éthane; les hydrocarbures cycloaliphatiques tels que le cyclohexane ou le méthylcyclohexane; les éthers aromatiques tels que l'anisole; les cétones telles que la méthylisobutylcétone; les nitriles tels que l'acétonitrile ou l'adiponitrile, les alcools tels que l'éthyl-2 hexanol et l'éthylène-glycol. Par solvant à point d'ébullition élevé, on entend un solvant à point d'ébullition supérieur à 70°C et de préférence au moins égal à 80°C.

La température à laquelle est effectuée le le chauffage de l'orthométhallypyrocatéchine ou de l'orthométhallyloxyphénol selon le procédé, doit être assez élevée pour permettre la transformation de l'orthométhallylpyrocatéchine ou de l'orthométhallyloxyphénol en DDHB. Elle doit cependant ne pas être trop élevée afin d'éviter la dégradation par la chaleur du DDHB formé. Avantageusement cette température est comprise entre 100 et 250°C et de préférence entre 170 et 230°C. Pour que l'eau et/ou le solvant organique soient liquides, on opère de préférence sous une pression suffisante pour rendre liquide les constituants présents dans le milieu réactionnel.

La durée nécessaire pour effectuer la transformation selon l'invention de l'orthométhallylpyrocatéchine ou de l'orthométhallyloxyphénol en DDHB dépend de la température utilisée, c'est à dire que cette durée est d'autant plus courte que la température est plus élevée. Avantageusement, cette durée est comprise entre une heure et quatre heures.

En fin de réaction, le DDHB obtenu est séparé par tout moyen connu en soi tel que par exemple par distillation. Toutefois, pour certaines utilisations, il peut ne pas être nécessaire d'isoler le DDHB et il suffit alors de le laisser dans le milieu réactionnel qui est lui-même mis en oeuvre de la manière souhaitée.

L'orthométhallyloxyphénol est un composé en soi connu dont la préparation a été décrite dans la demande de brevet français n° 2 255 279.

Les exemples suivants donnés à titre non limitatif illustrent l'invention et montrent comment elle peut être mise en oeuvre.

Exemple 1

Dans un autoclave, on charge 1,50 g d'orthométallyloxyphénol, 15 $cm^3$ de dodécane et 10 $cm^3$ d'eau. On purge l'autoclave à l'argon et on chauffe à 200°C pendant 2h45 mn et on laisse alors refroidir. On vide alors l'autoclave et on le rince à l'acétate d'éthyle (10 $cm^3$) qu'on joint à la masse réactionnelle brute. On décante et on extrait la phase aqueuse par deux fois à l'aide de 5 $cm^3$ d'acétate d'éthyle chaque fois. On joint ces extraits à la phase organique décantée dans laquelle on détermine par analyse chromatographique 1,075 g de DDHB et on observe que le mélange réactionnel ne contient plus d'orthométhallyloxyphénol.

Exprimés en pourcentage, les résultats sont les suivants:

— taux de transformation de l'orthométhallyloxyphénol mis en oeuvre: 100%
— rendement molaire en DDHB par rapport à l'orthométhallyloxyphénol: 71,6%.

Exemples 2 à 4

On opère comme à l'exemple 1 en faisant toutefois varier la quantité d'orthométhallyloxyphénol utilisée, le milieu rêactionnel, la durée et la température du chauffage.

Les résultats observés sont consignés dans le tableau ci-après dans lequel les termes suivants ont respectivement la signification suivante:

Monoéther: quantité d'orthométhallyloxyphénol utilisée
DDHB: quantité de dihydro-2,3 diméthyl-2,2 hydroxy-7 benzofurane obtenue
Rt (DDHB): rendement molaire en DDHB par rapport à l'orthométhallyloxyphénol.

Le taux de transformation de l'orthométhallyloxyphénol dans ces exemples était égal ou pratiquement égal à 100%.

| Exemple | Monoéther (en g) | Milieu réactionnel | Chauffage | | DDHB en g | Rt (DDHB) |
|---|---|---|---|---|---|---|
| | | | température | durée | | |
| 2 | 1, 473 | Dodécane ($15\ cm^3$) Eau ($10\ cm^3$) | 200 °C | 2 H | 1,044 | 71,3 % |
| 3 | 3, 0 | Eau ($9\ cm^3$) | 200 °C | 1 H25 | 2,075 | 69,1 % |
| 4 | 1, 50 | Toluène ($15\ cm^3$) Eau ($10\ cm^3$) | 200 °C | 2 H | 1,081 | 72,1 % |

Exemple 5

Dans un autoclave de 50 $cm^3$ en titane, on charge 1,1018 g d'orthométhallyloxyphénol et 10 $cm^3$ de cyclohexane. On chauffe 1h30mn à 200°C puis on refroidit.

L'analyse montre alors que l'orthométhallyloxyphénol a disparu du milieu réactionnel et qu'il s'est formé en majorité des méthallylpyrocatéchines.

On ajoute 5 $cm^3$ d'eau puis chauffe à nouveau à 200°C pendant 1h30mn puis refroidit: on a obtenu du DDHB avec un rendement de 70,5% par rapport à l'orthométhallyloxyphénol mis en oeuvre.

**Revendications**

1. Procédé de préparation de dihydro-2,3 diméthyl-2,2 hydroxy-7 benzofurane à partir d'orthométhallylpyrocatéchine caractérisé en ce que cette orthométhallylpyrocatéchine est chauffée en présence d'eau.

2. Procédé selon la revendication 1 caractérisé en ce que l'orthométhallylpyrocatéchine est obtenue par chauffage d'orthométhallyloxyphénol en présence d'eau et éventuellement d'un solvant organique inerte.

3. Procédé selon la revendication 1 caractérisé en ce que l'orthométhallylpyrocatéchine est obtenue par chauffage d'orthométhallyloxyphénol en l'absence d'eau, en présence d'un solvant organique inerte.

4. Procédé selon l'une des revendications 1 à 3, caractérisé en ce que le chauffage est effectué encore en présence d'un solvant organique inerte.

5. Procédé selon l'une des revendications 1 à 4, caractérisé en ce que le solvant organique a un point d'ébullition supérieur à 70°C.

6. Procédé selon l'une des revendications 1 à 5, caractérisé en ce que le solvant organique est un hydrocarbure aromatique ou un hydrocarbure aliphatique ou un hydrocarbure cycloaliphatique ou une cétone ou un nitrile ou un alcool.

7. Procédé selon la revendication 6, caractérisé en ce que le solvant est l'octane ou le dodécane ou le méthylcyclohexane ou le cyclohexane ou le toluène, ou les xylènes ou la méthylisobutylcétone.

8. Procédé selon l'une des revendications 1 à 7 caractérisé en ce que le rapport en volume solvant/eau mis en oeuvre est inférieur à 3.

9. Procédé selon l'une des revendications 1 à 8, caractérisé en ce que la température de réaction est comprise entre 100 et 250°C.

10. Procédé selon la revendication 9, caractérisé en ce que la température de réaction est comprise entre 170 et 230°C.

11. Procédé de préparation de dihydro-2,3 diméthyl-2,2 hydroxy-7 benzofurane selon l'une des revendications 1 à 10 caractérisé en ce que l'on chauffe de l'orthométhallylpyrocatéchine ou de l'orthométhallyloxyphénol en présence d'eau, le rapport pondéral eau/orthométhallyloxyphénol ou orthométhallylpyrocatéchine engagé étant compris entre 0,1 et 20.

12. Procédé selon l'une des revendications 1 à 11, caractérisé en ce que le rapport pondéral eau/orthométhallyloxyphénol ou orthométhallylpyrocatéchine engagé est compris entre 0,3 et 5.

**Patentansprüche**

1. Verfahren zur Herstellung von 2,3-Dihydro-2,2-dimethyl-7-hydroxybenzofuran ausgehend von o-Methallylbrenzcatechin, dadurch gekennzeichnet, dass das o-Methallylbrenzcatechin in Gegenwart von Wasser erhitzt wird.

2. Verfahren nach Anspрüch 1, dadurch gekennzeichnet, dass das o-Methallylbrenzcatechin durch Erhitzen von o-Methallyloxyphenol in Gegenwart von Wasser und gegebenfalls einem inerten organischen Lösungsmittel erhalten wird.

3. Verfahren nach Ansprüch 1, dadurch gekennzeichnet, dass das o-Methallylbrenzcatechin durch Erhitzen von o-Methallyloxyphenol ohne Wasser, in Gegenwart von einem inerten organischen Lösungsmittel erhalten wird.

4. Verfahren nach einem der Ansprüche 1 bis 3, dadurch gekennzeichnet, dass man zusätzlich in Gegenwart eines inerten organischen Lösungsmittels erhitzt.

5. Verfahren nach einem der Ansprüche 1 bis 4, dadurch gekennzeichnet, daß das organische Lösungsmittel einen Siedepunkt oberhalb 70°C aufweist.

6. Verfahren nach einem der Ansprüche 1 bis 5, dadurch gekennzeichnet, daß das organische Lösungsmittel ein aromatischer Kohlenwasserstoff, oder ein aliphatischer Kohlenwasserstoff, oder ein cycloaliphatischer Kohlenwasserstoff, oder ein Keton, oder ein Nitril, oder ein Alkohol ist.

7. Verfahren nach Anspruch 6, dadurch gekennzeichnet, daß das Lösungsmittel Octan oder Dodecan, oder Methylcyclohexan, oder Cyclohexan, oder Toluol, oder ein Xylol, oder Methylisobutylketon ist.

8. Verfahren nach einem der Ansprüche 1 bis 7, dadurch gekennzeichnet, daß das Volumenverhältnis von eingesetztem Lösungsmittel zu Wasser $<$ als 3 ist.

9. Verfahren nach einem der Ansprüche 1 bis 8, dadurch gekennzeichnet, daß die Reaktionstemperatur im Bereich von 100 bis 250°C liegt.

10. Verfahren nach Anspruch 9, dadurch gekennzeichnet, daß die Reaktionstemperatur im Bereich von 170 bis 230°C liegt.

11. Verfahren zur Herstellung von 2,3-Dihydro-2,2-dimethyl-7-hydroxybenzofuran nach einem der Ansprüche 1 bis 10, dadurch gekennzeichnet, daß man o-Methallylbrenzcatechin oder o-Methallyloxyphenol in Gegenwart von Wasser erhitzt, wobei das Gew.-Verhältnis von Wasser zu o-Methallyloxyphenol oder zu o-Methallylbrenzcatechin 0,1 bis 20 beträgt.

12. Verfahren nach einem der Ansprüche 1 bis 11, dadurch gekennzeichnet, daß das Gew.-Verhältnis von Wasser zu o-Methallyloxyphenol oder o-Methallylbrenzcatechin 0,3 bis 5 beträgt.

**Claims**

1. A process for the preparation of 2,3-dihydro-2,2-dimethyl-7-hydroxybenzofuran from ortho-methallylpyrocatechol, characterized in that this ortho-methallylpyrocatechol is heated in the presence of water.

2. A process according to claim 1, characterized in that the ortho-methallylpyrocatechol is prepared by heating orthomethallyloxyphenol in the presence of water and optionally of an inert organic solvent.

3. A process according to claim 1, characterized in that ortho-methallylpyrocatechol is prepared by heating ortho-methallyloxyphenol in the absence of water, in the presence of an inert organic solvent.

4. A process according to one of claims 1 to 3, characterized in that the heating is also carried out in the presence of an inert organic solvent.

5. A process according to one of claims 1 to 4, characterized in that the organic solvent has a b.p. above 70°C.

6. A process according to one of claims 1 to 5, characterized in that the organic solvent is an aromatic hydrocarbon, or an aliphatic hydrocarbon or a cycloaliphatic hydrocarbon or a ketone or a nitrile or an alcohol.

7. A process according to claim 6, characterized in that the solvent is octane or dodecane or methylcyclohexane or cyclohexane or toluene or xylenes or methyl isobutyl ketone.

8. A process according to one of claims 1 to 7, characterized in that the volume ratio solvent/water employed is less than 3.

9. A process according to one of claims 1 to 8, characterized in that the reaction temperature is between 100 and 250°C.

10. A process according to claim 9, characterized in that the reaction temperature is between 170 and 230°C.

11. A process for the preparation of 2,3-dihydro-2,2-dimethyl-7-hydroxybenzofuran, according to one of claims 1 to 10, characterized in that ortho-methallylpyrocatechol or ortho-methallyloxyphenol is heated in the presence of water, the weight ratio water/ortho-methallyloxyphenol or ortho-methallylpyrocatechol used being between 0.1 and 20.

12. A process according to one of claims 1 to 11, characterized in that the weight ratio water/ortho-methallyloxyphenol or ortho-methallylpyrocatechol used is between 0.3 and 5.